# FASCICULE DE BREVET EUROPEEN

(11) **EP 1 457 865 B1**
(45) Date de publication et mention de la délivrance du brevet: **08.11.2017**
(21) Numéro de dépôt: 03005615.4
(22) Date de dépôt: 12.03.2003
(51) Int. Cl.: G06F 3/041

(54) **SUBSTRAT À ÉLECTRODES TRANSPARENT ET SON PROCÉDÉ DE FABRICATION**
SUBSTRAT MIT UNSICHTBAREN ELEKTRODEN UND DESSEN HERSTELLUNGSVERFAHREN
SUBSTRATE WITH TRANSPARENT ELECTRODES AND CORRESPONDING FABRICATION PROCESS

(43) Date de publication de la demande: 15.09.2004
(73) Titulaire: ASULAB S.A., 2074 Marin (CH)
(72) Inventeur: Grupp, Joachim, 2073 Enges (CH); Poli, Gian-Carlo, 2206 Les Geneveys-sur-Coffrane (CH); Baroni, Pierre-Yves, 2013 Colombier (CH); Wagner, Estelle, 1024 Ecublens (CH); Hoffmann, Patrik, 1066 Epalinges (CH)
(74) Mandataire: Ravenel, Thierry Gérard Louis

(56) Documents cités:
- EP-A- 0 789 295
- EP-A- 1 087 286
- WO-A-01/88958
- US-B1- 6 414 728

## Description

La présente invention a pour objet un substrat transparent ayant au moins une face pourvue d'électrodes transparentes dont la structuration et l'agencement ne peuvent pas être perçus par l'utilisateur dans le domaine de longueurs d'ondes de la lumière visible.

L'invention concerne également des dispositifs comportant un ou plusieurs substrats à électrodes transparents dans lesquels les électrodes ont des rôles de commandes ou de collecteurs d'énergie, et plus particulièrement de tels dispositifs disposés au-dessus de l'affichage d'un appareil électronique sur lequel l'utilisateur doit pouvoir lire les informations dudit affichage, sans être gêné par la structuration et l'agencement des électrodes.

L'invention concerne également un procédé permettant d'effectuer avec une grande précision une structuration des électrodes sur un substrat transparent quelconque et une compensation optique entre les électrodes de façon à les rendre pratiquement invisibles.

Des solutions ont déjà été proposées pour que l'interface technique constituée par les électrodes reste la plus discrète possible et ne nuise pas à l'aspect esthétique de l'appareil électronique, notamment dans le cas d'une pièce d'horlogerie. On connaît par exemple des montres-bracelets dont la face intérieure de la glace comporte des électrodes permettant une commande tactile des fonctions horaires ou non horaires par effet capacitif ou résistif, comme cela est décrit de façon non limitative dans les brevets US 4,228,534, EP 0 674 247 et EP 1 207 439. La glace peut également être remplacée ou complétée par une cellule formée de deux substrats à électrodes transparents entre lesquels est placé un matériau actif, par exemple pour former une cellule photovoltaïque constituant la source d'énergie comme décrit dans le document WO 93/19479, ou pour former une cellule à cristaux liquides pouvant avoir un état transparent et un état permettant d'afficher sur demande des informations complémentaires ou différentes de celles affichées sur un cadran sous-jacent, comme décrit dans le document WO 99/32945.

Pour réaliser les électrodes on utilise de façon connue des oxydes conducteurs transparents (TCO) c'est-à-dire des matériaux qui sont à la fois bon conducteurs et transparents dans le visible, tels que l'oxyde d'étain et d'indium (ITO), In₂O₃ ou SnO₂ dopé à l'antimoine. Ces dépôts, de l'ordre de 50 à 100nm, sont effectués directement sur le substrat transparent ou sur une couche intermédiaire par un grand nombre de techniques connues telles que la pulvérisation, l'évaporation, la technique sol-gel, ainsi que des techniques de dépôt en phase vapeur (CVD) dont on retiendra particulièrement le dépôt assisté par laser (LICVD). En ce qui concerne la structuration des électrodes, il existe également différentes méthodes connues mises en oeuvre, en utilisant au moins un masque correspondant au contour des électrodes, soit lors du dépôt du TCO par cristallisation localisée d'un film sol-gel par irradiation avec un laser UV, soit en effectuant sur un film continu de TCO une attaque chimique ou une ablation localisée en irradiant avec un laser UV à une fluence suffisante. La nature du substrat transparent, verre ou plastique, est évidemment déterminante du point de vue technique et économique pour le choix du processus à appliquer. La cristallisation localisée d'un film sol-gel par laser UV n'est, par exemple, pas applicable à un substrat plastique, tel que le PMMA, car elle relève d'un processus photothermique.

En incidence normale, un rayon lumineux traversant le TCO d'indice de réfraction n₁ et le substrat d'incidence de réfraction traversant n₀, ou seulement le substrat n'est pas dévié de sorte que les électrodes sont invisibles. Par contre en incidence oblique, en raison des valeurs différentes de n₀ et n₁ le trajet lumineux est modifié, de sorte que le contour des électrodes devient visible. De façon évidente, il suffit de combler ce vide par un matériau de remplissage non conducteur ayant un indice de réfraction n₂ proche de n₁. Des matériaux de ce type sont généralement des oxydes transparents non conducteurs (NCTO) tels que SiO₂ ou TiO₂. Divers procédés ont été utilisés pour atteindre ce but. Ils ne sont toutefois pas satisfaisants en ce que le matériau de remplissage peut former des bourrelets en dehors de la zone de remplissage, ou inversement des dépressions susceptibles de modifier le trajet des rayons lumineux rendant encore visible le contour des électrodes, comme cela sera expliqué plus loin.

Les brevets US 6 414 728 et EP 0 789 295 illustrent des dispositifs comprenant un substrat à électrodes dans lesquels deux indices de réfraction doivent avoir des valeurs les plus proches possibles pour améliorer la qualité de l'image. Dans le brevet US 6 414 728, concernant une cellule d'affichage, il s'agit de l'indice de réfraction des substrats transparents et de l'indice de réfraction de la couche PDLC disposée entre les deux substrats. Dans le brevet EP 0 789 295 il s'agit de l'indice de réfraction des substrats transparentes formant une cellule d'affichage et de l'indice de réfraction d'une colle transparente permettant de fixer un écran tactile au dessus de la cellule. En aucun cas l'indice de réfraction du matériau constituant les électrodes n'est pris en considération.

L'invention a donc pour objet un substrat transparent supportant des électrodes dont le contour est invisible à un usager observant ledit substrat sous une incidence quelconque.

A cet effet l'invention a pour objet un substrat à électrodes transparent formé d'un matériau transparent d'indice de réfraction n₀ sur lequel est déposé un film d'un matériau conducteur transparent d'épaisseur e₁ et d'indice de réfraction n₁. Le film est structuré pour former un ensemble d'électrodes dont les contours délimitent des espaces isolants destinés à être garnis avec un matériau diélectrique transparent d'épaisseur e₂ et d'indice de réfraction n₂. Le substrat est caractérisé en ce que les valeurs des paramètres e₁, e₂, n₁, n₂ sont telles que les épaisseurs respectives du matériau conducteur et du matériau diélectrique sont inversement proportionnelles aux valeurs des indices de réfraction desdits matériaux en formant à leur jonction ni dépression, ni bourrelet.

La matériau conducteur transparent est de préférence un oxyde conducteur transparent (TCO) tel qu'un oxyde d'étain et d'indium (ITO), In₂O₃ ou SnO₂ dopé par Sb. Le matériau diélectrique est de préférence un oxyde transparent non conducteur (NCTO) tel que TiO₂ et SiO₂.

Le matériau transparent formant la base du substrat peut être du verre ou un matériau plastique transparent tel que le polyméthyl méthacrylate (PMMA) ou le polycarbonate (PC). Lorsqu'il s'agit d'un matériau plastique on interpose de préférence, entre le substrat et le film TCO, une couche intermédiaire d'un matériau transparent et dur, tel qu'une résine incorporant SiO₂.

Le substrat tel que décrit ci-dessus peut en outre être revêtu d'un film protecteur vis-à-vis des agressions mécaniques, par exemple lors des manipulations pour monter ledit substrat dans un appareil électronique, ou chimiques lorsqu'il constitue une plaque de fermeture d'une cellule à cristaux liquides ou d'une cellule photovoltaïque. Le procédé permettant d'obtenir ledit substrat à électrodes transparent consiste fondamentalement à
- placer le substrat, préalablement revêtu d'un film continu de TCO, dans une enceinte fermée comportant une fenêtre transparente au rayonnement UV, une amenée de gaz et une sortie de pompage dudit gaz;
- par la fenêtre de l'enceinte, effectuer une première irradiation avec une source UV à travers un masque dont les parties transparentes au rayonnement UV correspondent aux espaces isolants à former dans le TCO, le rayonnement UV ayant des caractéristiques ajustées en fonction de la nature et de l'épaisseur du TCO pour provoquer son arrachage dans les zones irradiées et former lesdits espaces isolants;
- introduire dans l'enceinte un gaz précurseur du NCTO, et avec la même source UV et le même masque effectuer une deuxième irradiation en adaptant les caractéristiques du rayonnement à la nature du NCTO pour provoquer un dépôt d'épaisseur e₂ dans les espaces isolants, et
- pomper le gaz précurseur et sortir le substrat à électrodes transparent de l'enceinte.

La source à rayonnement UV est constituée par un laser, par exemple un laser excimer de 248 nm à impulsions courtes ou de 308 nm à impulsions longues, les caractéristiques du rayonnement UV étant la fluence, la fréquence et le nombre d'impulsions, tous ces paramètres étant déterminants pour éliminer le TCO sans dégradation du substrat et pour contrôler l'épaisseur du dépôt de NCTO, comme on le verra dans la description détaillée qui suit.

D'autres caractéristiques et avantages de la présente invention apparaîtront dans la description suivante donnée à titre illustratif et non limitatif, en référence aux dessins annexés dans lesquels :
- les figures 1A, 1B et 1C représentent un procédé de l'art antérieur et le substrat à électrodes ainsi obtenu;
- la figure 2 représente un autre substrat à électrodes obtenu avec un autre procédé de l'art antérieur;
- la figure 3 est une représentation schématique d'un dispositif permettant d'obtenir un substrat à électrodes transparent selon l'invention;
- les figures 4A, 4B et 4C représentent les étapes de fabrication d'un substrat à électrodes transparent selon l'invention, et
- les figures 5A à 5E représentent les étapes de fabrication d'un autre substrat à électrodes transparent selon l'invention.

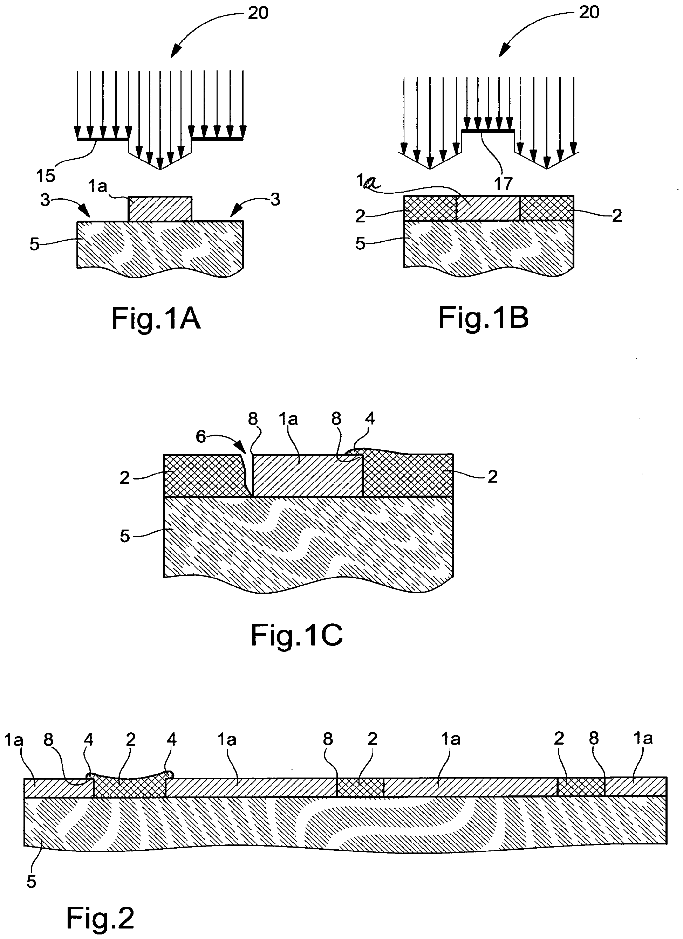

Les figures 1A et 1B représentent schématiquement un procédé de l'art antérieur faisant appel à la technologie dite LICVD (Light Induced Chemical Vapor Déposition) telle que décrite, par exemple, par Wagner E. (STI, Micro-engineering 2003, EPFL : Lausanne). Dans une première étape, représentée à la figure 1A, un substrat transparent 5 est placé dans une enceinte (non représentée) dans laquelle on introduit un gaz précurseur 20, par exemple Sn (CH₃)₄ ou Sn Cl₄ pour le dépôt d'un film de SnO₂, oxyde transparent conducteur (TCO). Ce dépôt est effectué par LICVD en irradiant à travers un premier masque 15 dont la conformation en transparence UV correspond à la configuration à obtenir pour les électrodes, séparées par des espaces isolants 3.

Dans une deuxième étape représentée à la figure 1B, on remplace le premier masque 15 par un deuxième masque 17 ayant une fenêtre transparente aux rayons UV complémentaire de celle du premier masque. La figure 1C montre les défauts qu'on peut observer lorsque les deux masques ne sont pas superposés de façon rigoureusement complémentaire. Il peut se produire, soit des bourrelets 4 de NCTO 2, soit au contraire des dépressions 6, qui vont modifier par endroits le trajet optique, et donc rendre visibles certaines portions du contour des électrodes.

Des défauts du même type peuvent être observés en garnissant de NCTO 2 les espaces isolants 3 par la technique bien connue de "lift-off". On peut alors observer des bourrelets 4 sur les deux bords 8 de l'espace isolant 3, comme représenté à la figure 2.

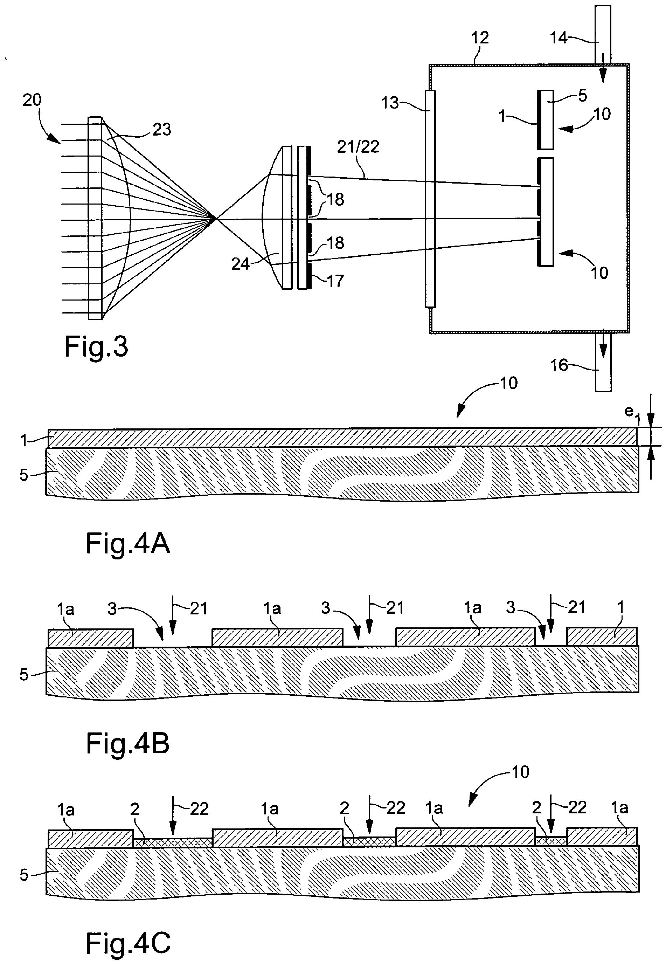

En se référant maintenant aux figures 3, 4A, 4B et 4C, on décrit ci-après comment l'invention permet d'effectuer une compensation optique parfaite rendant les électrodes pratiquement invisibles à l'oeil nu.

La figure 3 représente schématiquement le dispositif qui comporte fondamentalement une source 20 de rayonnement UV constituée par une source laser, une optique comprenant une lentille convergente 23 et une lentille divergente 24 permettant de diminuer la taille du faisceau laser afin d'en augmenter la fluence, un masque 17 comportant des zones 18 transparentes au rayonnement UV, et une enceinte 12. L'enceinte 12 comporte une fenêtre 13 transparente au rayonnement UV, une amenée 14 de gaz précurseurs et une sortie 16 de pompage desdits gaz précurseurs. L'enceinte 12 peut également comporter une amenée supplémentaire (non représentée) d'un gaz vecteur du gaz précurseur. A l'intérieur de l'enceinte 12 on a placé un substrat 10 qui, dans cet exemple comporte une base transparente 5 et qui est déjà revêtue d'un film 1 en TCO. Il serait évidemment possible, mais d'un intérêt économique moindre, de former le film TCO directement dans l'enceinte 12, par LICVD comme indiqué en préambule.

La source de rayonnement UV est constituée par un laser excimer, tel qu'un laser XeCl (308 nm) à impulsion longue (250 ns) fournissant une énergie maximale de 150 mJ par impulsion avec un faisceau rectangulaire de 1,9 x 2,4 cm², ou un laser KrF (248 nm) à impulsion courte (20 ns) fournissant une énergie maximale de 180 mJ par impulsion avec un faisceau rectangulaire de 1,5 x 4 cm³. D'autres lasers excimer peuvent évidemment être utilisés.

La figure 4A représente à titre d'exemple un substrat 5 en verre sur lequel a été déposé un film 1 continu de ITO d'épaisseur e₁ = 70 nm. Ce substrat est introduit dans l'enceinte 12 et soumis à travers le masque 17 à un rayonnement UV émis par un laser excimer à 308 nm à impulsion longue. Dans une première étape représentée à la figure 4B, on ajuste les caractéristiques du faisceau 21 du laser 20 avec une fluence de ≥ 300 mJ/cm² 140 mJ/cm² et 500 impulsions à 5 Hz permettant d'éliminer l'ITO dans les espaces isolants 3 correspondant aux espaces transparents 18 ménagés dans le masque 17. Dans une deuxième étape représentée à la figure 4C, correspondant au processus en cours de réalisation, en conservant exactement le même masque 17, et sans déplacer le substrat 10, on introduit dans l'enceinte 12 du tétraisoproxide de titane (TTIP) à 97 % comme gaz précurseur pour effectuer un dépôt de TiO₂ 2 dans les conditions préconisées par Wagner E. (déjà cité) et on ajuste la fluence à 60 mJ/cm² avec 4000 impulsions à 20 Hz pour obtenir l'épaisseur e₂ de TiO₂ sensiblement égale à l'épaisseur e₁ de ITO. En effet, dans les conditions de dépôt, TiO₂ a un indice de réfraction n₂ mesuré de 2,05, soit sensiblement l'indice de réfraction n₁ de l'ITO qui a pour valeur 2. Cette phase de dépôt est de préférence effectuée en introduisant dans l'enceinte 12 également un gaz vecteur, tel que l'oxygène, l'azote ou un mélange des deux, permettant d'ajuster la pression partielle en précurseur et de rendre le dépôt plus homogène. Dans cet exemple, il serait possible de remplacer le substrat en verre par un substrat en matériau plastique tel que le PMMA, à condition de ne pas dépasser la fluence seuil de dégradation dudit matériau, tant en ablation de l'ITO qu'en dépôt de TiO₂.

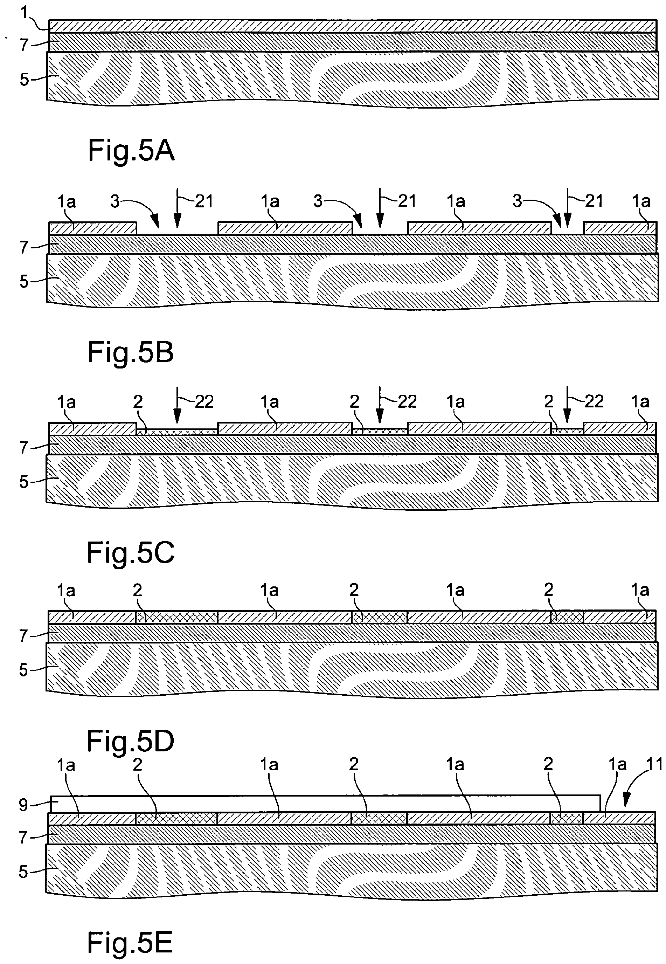

Il est toutefois apparu préférable, comme expliqué en référence aux figures 5A à 5E, de déposer sur le support en PMMA une couche intermédiaire 7 transparente et dure (hardcoating) ayant au moins 20 µm d'épaisseur et sur laquelle sera déposé un film d'ITO 1 d'environ 70 µm d'épaisseur comme représenté à la figure 5A, dans laquelle les épaisseurs relatives des couches ne sont pas respectées. Cette couche intermédiaire, qui doit également avoir une fluence seuil de dégradation supérieure à celle du PMMA est par exemple composée d'une résine à base de SiO₂. Elle joue également un rôle mécanique en permettant à la couche d'ITO de mieux adhérer et de ne pas se dégrader lors des déformations du PMMA dues aux élévations de température. La figure 5B représente le substrat 5 après conformation des électrodes 1a séparées par les espaces isolants 3. Dans cet exemple, l'ablation du film d'ITO a été effectuée au moyen d'un laser excimer à 248 nm à impulsions courtes avec une fluence de 80 mJ/cm² et 10 impulsions à 5 Hz. Dans l'étape représentée à la figure 5C, on a commencé d'effectuer le dépôt de TiO₂ 2 dans les même conditions que pour le premier exemple, mais en modifiant les caractéristiques du laser de sorte à avoir une fluence de 6 mJ/cm² avec 10000 impulsions à 5 Hz. La figure 5D représente le même substrat en fin de dépôt de TiO₂. Comme on peut le voir, le procédé dans lequel, ni le masque 17, ni le substrat 10 ne sont déplacés, permet d'avoir un dépôt de TiO₂ ne formant ni bourrelet 4, ni dépression 6, ce qui permet de rendre les électrodes 1a pratiquement invisible.

La figure 5C représente une étape optionnelle dans laquelle le substrat 10 reste dans l'enceinte 12 sans déplacement mais où le masque 17 est remplacé par un masque correspondant au contour utile du substrat à électrodes transparent. On introduit alors dans l'enceinte, en réglant à nouveau les caractéristiques du laser, un gaz précurseur permettant le dépôt d'un film protecteur 9, formé par exemple d'un dépôt de SiO₂ et TiO₂, pouvant éventuellement être modifié pour avoir des propriétés anti-reflets. Ce film 9 permet en outre de délimiter sélectivement les zones de contact 11 des électrodes 1a.

Dans ce deuxième exemple, il est bien évident que le PMMA peut être remplacé par un autre matériau plastique transparent, tel que le polycarbonate (PC) en adaptant si nécessaire les caractéristiques du faisceau laser.

Le substrat à électrodes transparent qui vient d'être décrit a de nombreuses applications, non seulement au niveau d'un écran tactile, d'une cellule à cristaux liquide, ou d'une cellule photovoltaïque, mais aussi dans d'autres applications à la portée de l'homme de l'art sans sortir du cadre de la présente invention.

## Revendications

1. Substrat à électrodes transparent formé d'un matériau transparent sur lequel est déposé un film (1) d'un matériau conducteur transparent d'épaisseur e₁ et d'indice de réfraction n₁, ledit film (1) étant structuré pour former un ensemble d'électrodes (1a) dont les contours (8) délimitent des espaces isolants (3), lesdites électrodes (1a) ayant près du bord du substrat (5) des zones de contact (11) connectables à un appareil électronique, les espaces isolants (3) étant garnis avec un matériau diélectrique transparent d'épaisseur e₂ et d'indice de réfraction n₂, **caractérisé en ce que** les épaisseurs respectives du matériau conducteur et du matériau diélectrique sont inversement proportionnelles aux valeurs des indices de réfraction desdits matériaux et **en ce que** ledit matériau diélectrique ne forme ni dépression, ni bourrelet au niveau du contour (8) des électrodes.

2. Substrat selon la revendication 1, **caractérisé en ce qu'**une couche (7) d'un matériau dur additionnel, transparent non conducteur et d'épaisseur uniforme est en outre interposé entre le substrat (5) et les électrodes (1a).

3. Substrat selon la revendication 2, **caractérisé en ce que** la couche intermédiaire dure (7) est composée d'une résine incorporant du SiO₂ ayant une épaisseur au moins égale à 20µm.

4. Substrat selon la revendication 2, **caractérisé en ce que** le substrat (5) est réalisé en un matériau plastique choisi parmi le polyméthylène méthacrylate (PMMA) et le polycarbonate (PC).

5. Substrat selon la revendication 1, **caractérisé en ce que** les électrodes (1a) et le matériau diélectrique garnissant les espaces isolants sont en outre recouverts d'un film protecteur (9) pouvant également avoir des propriétés anti-reflets.

6. Substrat selon la revendication 5, **caractérisé en ce que** le film protecteur (9) ne recouvre pas les zones de contact (11) des électrodes (1a).

7. Substrat selon la revendication 1, **caractérisé en ce que** le matériau conducteur transparent formant les électrodes (1a) est un oxyde conducteur transparent (TCO) choisi parmi un oxyde d'étain et d'indium (ITO), In₂O₃ et SnO₂ dopé par Sb.

8. Substrat selon la revendication 1, **caractérisé en ce que** le matériau diélectrique garnissant les espaces (3) entre les électrodes (1a) est un oxyde transparent non conducteur (NCTO) choisi parmi TiO₂ et SiO₂.

9. Substrat selon la revendication 1, **caractérisé en ce que** l'épaisseur e₁ du matériau conducteur transparent est comprise entre 50 et 100nm, de préférence entre 65 et 75nm.

10. Substrat selon la revendication 1, **caractérisé en ce qu'**il constitue un écran tactile capacitif ou résistif de commande de l'appareil électronique auquel il est associé.

11. Substrat selon la revendication 1, **caractérisé en ce qu'**il constitue au moins une plaque de fermeture d'une cellule d'affichage à cristaux liquides ou d'une cellule photovoltaïque associé à l'appareil électronique.

12. Procédé de fabrication d'un substrat à électrodes transparent comportant un substrat transparent (5) sur lequel sont structurées des électrodes (1a) en un oxyde conducteur transparent (TCO) d'épaisseur e₁ et d'indice de réfaction n₁, les espaces diélectriques compris (3) entre les électrodes (1a) étant comblés par un oxyde transparent non conducteur (NCTO) d'épaisseur e₂ et d'indice de réfraction n₂, **caractérisé en ce que** les épaisseurs respectives du matériau conducteur et du matériau diélectrique sont inversement proportionnelles aux valeurs des indices de réfraction desdits matériaux, et **en ce que** le procédé consiste en les étapes suivantes :
- placer le substrat (5), préalablement revêtu d'un film continu (1) de TCO, éventuellement déposé sur une couche intermédiaire (7) d'un matériau dur transparent, dans une enceinte fermée (12) comportant une fenêtre transparente (13) au rayonnement UV, une amenée de gaz (14) et une sortie de pompage (16) dudit gaz;
- par la fenêtre (13) de l'enceinte (12), effectuer une première irradiation avec une source UV à travers un masque (17) dont les parties transparentes (18) au rayonnement UV correspondent aux espaces diélectriques (3), le rayonnement UV ayant des caractéristiques ajustées en fonction de la nature et de l'épaisseur du TCO pour provoquer son arrachage dans les zones irradiées et former les espaces diélectriques (3);
- introduire dans l'enceinte (12) un gaz précurseur du NCTO, et avec la même source UV et le même masque (17) effectuer une deuxième irradiation en adaptant les caractéristiques du rayonnement UV à la nature du NCTO pour provoquer une déposition d'épaisseur e₂ dans les espaces diélectriques (3), et
- pomper le gaz précurseur et sortir le substrat à électrodes transparent de l'enceinte (12).

13. Procédé selon la revendication 12, **caractérisé en ce que** les caractéristiques de fluence et de fréquence des première et deuxième irradiations sont ajustées de façon à ce que les épaisseurs de TCO et de NCTO soient inversement proportionnelles à leurs indices de réfraction.

14. Procédé selon la revendication 12, **caractérisé en ce que** le gaz précurseur est introduit dans l'enceinte (12) en même temps qu'un gaz vecteur.

15. Procédé selon la revendication 12, **caractérisé en ce qu'**il comporte une étape supplémentaire consistant à effectuer la déposition d'un film protecteur (9), éventuellement anti-reflet, à partir d'un autre gaz précurseur, avec la même source UV mais en remplaçant le masque utilisé pour la structuration par un masque correspondant à la surface utile souhaitée pour le substrat à électrodes transparent, en laissant apparentes les zones de contact (11) des électrodes (1a).

16. Procédé selon la revendication 12, **caractérisé en ce que** la source UV est constituée par un laser excimer de 248 nm à pulses courts ou de 308 nm à pulses longs.

17. Procédé selon la revendication 12, **caractérisé en ce que** le TCO est choisi parmi un oxyde d'étain et d'indium (ITO), In₂O₃ et SnO₂ dopé par Sb.

18. Procédé selon la revendication 12, **caractérisé en ce que** le NCTO est choisi parmi TiO₂ et SiO₂.

19. Procédé selon la revendication 18, **caractérisé en ce que** TiO₂ est obtenu à partir de tétra-isopropoxyde de titane (TTIP) comme gaz précurseur.

## Patentansprüche

1. Lichtdurchlässiges Elektrodensubstrat, das aus einem lichtdurchlässigen Material gebildet ist, auf dem ein Film (1) aus einem lichtdurchlässigen Leitermaterial mit einer Dicke e₁ und einem Brechungsindex n₁ abgelagert ist, wobei der Film (1) strukturiert ist, um eine Elektrodenanordnung (1a) zu bilden, deren Umrisse (8) isolierende Zwischenräume (3) begrenzen, wobei die Elektroden (1a) in der Nähe des Randes des Substrats (5) Kontaktbereiche (11) aufweisen, die mit einer elektronischen Vorrichtung verbindbar sind, wobei die isolierenden Zwischenräume (3) mit einem lichtdurchlässigen dielektrischen Material mit Dicke e₂ und Brechungsindex n₂ versehen sind, **dadurch gekennzeichnet, dass** die jeweiligen Dicken des Leitermaterials und des dielektrischen Materials zu den Werten der Brechungsindizes der Materialien umgekehrt proportional sind und dass das dielektrische Material im Bereich des Umrisses (8) der Elektroden weder eine Vertiefung noch einen Wulst bildet.

2. Substrat nach Anspruch 1, **dadurch gekennzeichnet, dass** außerdem eine Schicht (7) aus einem zusätzlichen harten, lichtdurchlässigen, nichtleitenden Material mit gleichmäßiger Dicke zwischen das Substrat (5) und die Elektroden (1a) eingefügt ist.

3. Substrat nach Anspruch 2, **dadurch gekennzeichnet, dass** die harte Zwischenschicht (7) aus einem Harz gebildet ist, das SiO₂ enthält, und eine Dicke hat, die mindestens gleich 20 µm ist.

4. Substrat nach Anspruch 2, **dadurch gekennzeichnet, dass** das Substrat (5) aus einem Kunststoff hergestellt ist, der aus Polyethylen-Methacrylat (PMMA) und Polycarbonat (PC) gewählt ist.

5. Substrat nach Anspruch 1, **dadurch gekennzeichnet, dass** die Elektroden (1a) und das dielektrische Material, mit dem die isolierenden Zwischenräume versehen sind, ferner mit einem Schutzfilm (9) bedeckt sind, der darüber hinaus antireflektierende Eigenschaften haben kann.

6. Substrat nach Anspruch 5, **dadurch gekennzeichnet, dass** der Schutzfilm (9) die Kontaktzonen (11) der Elektroden (1a) nicht bedeckt.

7. Substrat nach Anspruch 1, **dadurch gekennzeichnet, dass** das lichtdurchlässige Leitermaterial, das die Elektroden (1a) bildet, ein lichtdurchlässiges leitendes Oxid (TCO) ist, das aus Indiumzinnoxid (ITO), In₂O₃ und SnO₂ dotiert mit Sb gewählt ist.

8. Substrat nach Anspruch 1, **dadurch gekennzeichnet, dass** das dielektrische Material, mit dem die Zwischenräume (3) zwischen den Elektroden (1a) versehen sind, ein nichtleitendes, lichtdurchlässiges Oxid (NCTO) ist, das aus TiO₂ und SiO₂ gewählt ist.

9. Substrat nach Anspruch 1, **dadurch gekennzeichnet, dass** die Dicke e₁ des lichtdurchlässigen Leitermaterials im Bereich von 50 bis 100 nm, vorzugsweise im Bereich von 65 bis 75 nm, liegt.

10. Substrat nach Anspruch 1, **dadurch gekennzeichnet, dass** es einen kapazitiven oder resistiven Touchscreen für die Steuerung des elektronischen Geräts, dem es zugeordnet ist, bildet.

11. Substrat nach Anspruch 1, **dadurch gekennzeichnet, dass** es wenigstens eine Platte zum Verschließen einer Flüssigkristallanzeigezelle oder einer Photovoltaikzelle, die dem elektronischen Gerät zugeordnet ist, bildet.

12. Verfahren zum Herstellen eines lichtdurchlässigen Elektrodensubstrats, das ein lichtdurchlässiges Substrat (5) umfasst, auf dem Elektroden (1a) aus einem lichtdurchlässigen leitenden Oxid (TCO) mit einer Dicke e₁ und einem Brechungsindex n₁ strukturiert sind, wobei die dielektrischen Zwischenräume (3) zwischen den Elektroden (1a) mit einem nichtleitenden, lichtdurchlässigen Oxid (NCTO) mit einer Dicke e₂ und einem Brechungsindex n₂ gefüllt sind,
**dadurch gekennzeichnet, dass**
die jeweiligen Dicken des Leitermaterials und des dielektrischen Materials zu den Werten der Brechungsindizes der Materialien umgekehrt proportional sind und dass das Verfahren auf den folgenden Schritten beruht:
- Anordnen des Substrats (5), das vorher mit einem ununterbrochenen TCO-Film (1) beschichtet worden ist, der gegebenenfalls auf einer Zwischenschicht (7) aus einem lichtdurchlässigen, harten Material abgelagert ist, in einen geschlossenen Behälter (12), der ein für UV-Strahlung durchlässiges Fenster (13), eine Gaszuführung (14) und einen Pumpausgang (16) für das Gas aufweist;
- Ausführen einer ersten Bestrahlung durch das Fenster (13) des Behälters (12) mit einer UV-Quelle durch eine Maske (17) hindurch, deren für UV-Strahlung durchlässigen Teile (18) den dielektrischen Zwischenräumen (3) entsprechen, wobei die UV-Strahlung Eigenschaften hat, die in Abhängigkeit von der Art und der Dicke der TCO eingestellt sind, um ihr Abheben in den bestrahlten Zonen zu bewirken und um die dielektrischen Zwischenräume (3) zu bilden;
- Einleiten in den Behälter (12) eines Vorläufergases des NCTO und Ausführen einer zweiten Bestrahlung mit derselben UV-Quelle und derselben Maske (17) unter Anpassung der Eigenschaften der UV-Strahlung an die Art des NCTO, um eine Ablagerung mit Dicke e₂ in den dielektrischen Zwischenräumen (3) hervorzurufen, und
- Abpumpen des Vorläufergases und Entnehmen des lichtdurchlässigen Elektrodensubstrats aus dem Behälter (12).

13. Verfahren nach Anspruch 12, **dadurch gekennzeichnet, dass** die Fluenz- und Frequenzeigenschaften der ersten und zweiten Bestrahlung in der Weise eingestellt sind, dass die Dicken des TCO und des NCTO zu ihren Brechungsindizes umgekehrt proportional sind.

14. Verfahren nach Anspruch 12, **dadurch gekennzeichnet, dass** das Vorläufergas in den Behälter (12) gleichzeitig mit einem Trägergas eingeleitet wird.

15. Verfahren nach Anspruch 12, **dadurch gekennzeichnet, dass** es einen zusätzlichen Schritt umfasst, der darin besteht, die Ablagerung eines gegebenenfalls antireflektierenden Schutzfilms (9) anhand eines anderen Vorläufergases mit derselben UV-Quelle, jedoch unter Ersetzung der für die Strukturierung verwendeten Maske durch eine Maske, die der für das lichtdurchlässige Elektrodensubstrat gewünschten Nutzoberfläche entspricht, auszuführen, wobei die Kontaktbereiche (11) der Elektroden (1a) sichtbar gelassen werden.

16. Verfahren nach Anspruch 12, **dadurch gekennzeichnet, dass** die UV-Quelle durch einen Excimer-Laser bei 248 nm mit kurzen Impulsen oder bei 308 nm mit langen Pulsen gebildet ist.

17. Verfahren nach Anspruch 12, **dadurch gekennzeichnet, dass** das TCO aus einem Indiumzinnoxid (ITO), In₂O₃ und SnO₂ dotiert mit Sb gewählt ist.

18. Verfahren Anspruch 12, **dadurch gekennzeichnet, dass** das NCTO aus TiO₂ und SiO₂ gewählt ist.

19. Verfahren nach Anspruch 18, **dadurch gekennzeichnet, dass** das TCO anhand von Tetraisopropyltitanat (TTIP) als Vorläufergas erhalten wird.

## Claims

1. A substrate with transparent electrodes and formed of a transparent material onto which is deposited a film (1) of a transparent conductive material of thickness e₁ and of refractive index n₁, said film (1) being structured to form a set of electrodes (1a) whose contours (8) delimit insulating spaces (3), said electrodes (1a) having contact areas (11) close to the edge of the substrate (5) that can be connected to an electronic device, the insulating spaces (3) being filled with a transparent dielectric material of thickness e₂ and of refractive index n₂, **characterized in that** the respective thicknesses of the conductive material and of the dielectric material are inversely proportional to the values of the refractive indices of said materials, and **in that** said dielectric material forms neither depressions, nor beads at the contour (8) of the electrodes.

2. A substrate according to claim 1, **characterised in that** a layer (7) of uniform thickness of an additional hard, transparent, non-conductive material is disposed between the substrate (5) and the electrodes (1a).

3. A substrate according to claim 2, **characterised in that** the hard intermediate layer (7) consists of a resin incorporating SiO₂ and at least 20 µm thick.

4. A substrate according to claim 2, **characterised in that** the substrate (5) is made from a plastics material selected from polymethylmethacrylate (PMMA) and polycarbonate (PC).

5. A substrate according to claim 1, **characterised in that** the electrodes (1a) and the dielectric material filling the insulating spaces are further covered with a protective film (9) that may also have anti-reflection properties.

6. A substrate according to claim 5, **characterised in that** the protective film (9) does not cover the contact areas (11) of the electrodes (1a).

7. A substrate according to claim 1, **characterised in that** the transparent conductive material forming the electrodes (1a) is a transparent conductive oxide (TCO) selected from indium and tin oxide (ITO), In₂O₃ and SnO₂ doped with Sb.

8. A substrate according to claim 1, **characterised in that** the dielectric material filling the spaces (3) between the electrodes (1a) is a non-conductive transparent oxide (NCTO) selected from TiO₂ and SiO₂.

9. A substrate according to claim 1, **characterised in that** the thickness e₁ of the transparent conductive material is from 50 to 100 nm and preferably from 65 to 75 nm.

10. A substrate according to claim 1, **characterised in that** it constitutes a capacitive or resistive touch-sensitive screen for controlling the associated electronic device.

11. A substrate according to claim 1, **characterised in that** it constitutes at least one closure plate of a liquid crystal display cell or a photovoltaic cell associated with the electronic device.

12. A method of fabricating a substrate with transparent electrodes comprising a transparent substrate (5) on which are structured electrodes (1a) of a transparent conductive oxide (TCO) of thickness e₁ and of refractive index n₁, the dielectric spaces (3) between the electrodes (1a) being filled with a non-conductive transparent oxide (NCTO) of thickness e₂ and of refractive index n₂, **characterized in that**:
- the respective thicknesses of the conductive material and of the dielectric material are inversely proportional to the values of the refractive indices of said materials,
and **in that** the method comprises the following steps:
- placing the substrate (5), previously coated with a continuous film (1) of TCO, possibly deposited onto an intermediate layer (7) of a hard transparent material, in a closed enclosure (12) having a window (13) transparent to UV radiation, a gas inlet (14) and an outlet (16) from which said gas is pumped;
- effecting through the window (13) of the enclosure (12), a first irradiation by a UV source through a mask (17) having portions (18) transparent to UV radiation that correspond to the dielectric spaces (3), the characteristics of the UV radiation being adjusted as a function of the nature and the thickness of the TCO to cause the tearing thereof it in the irradiated areas and form the dielectric spaces (3);
- introducing a precursor gas of the NCTO into the enclosure (12) and, using the same UV source and the same mask (17), effecting a second irradiation with the characteristics of the UV radiation adapted to the nature of the NCTO to produce a deposit of thickness e₂ in the dielectric spaces (3); and
- pumping out the precursor gas and removing the substrate with transparent electrodes from the enclosure (12).

13. A method according to claim 12, **characterised in that** the fluence and the frequency characteristics of the first and second irradiations are adjusted so that the thicknesses of TCO and NCTO are inversely proportional to their refractive indices.

14. A method according to claim 12, **characterised in that** the precursor gas is introduced into the enclosure (12) at the same time as a vector gas.

15. A method according to claim 12, **characterised in that** it includes an additional step of depositing a protective film (9), possibly having anti-reflection properties, from another precursor gas, using the same UV source but replacing the mask used to structure the electrodes with a mask corresponding to the required active surface of the substrate with transparent electrodes, leaving exposed the contact areas (11) of the electrodes (1a).

16. A method according to claim 12, **characterised in that** the UV source is a 248 nm excimer laser emitting short pulses or a 308 nm excimer laser emitting long pulses.

17. A method according to claim 12, **characterised in that** the TCO is selected from indium and tin oxide (ITO), In₂O₃, and Sn02 doped with Sb.

18. A method according to claim 12, **characterised in that** the NCTO is selected from TiO₂ and SiO₂.

19. A method according to claim 18, **characterised in that** the TiO₂ is obtained from titanium tetraisopropoxide (TTIP) precursor gas.
